# EUROPEAN PATENT SPECIFICATION

(11) **EP 3 621 523 B1**
(45) Date of publication and mention of the grant of the patent: **31.03.2021**
(21) Application number: 18730190.8
(22) Date of filing: 10.05.2018
(51) Int. Cl.: A61B 6/00

(54) **SOFTWARE SYNCHRONIZATION FOR BEDSIDE DYNAMIC IMAGING**
SOFTWARESYNCHRONISATION FÜR BETTSEITIGE DYNAMISCHE BILDGEBUNG
SYNCHRONISATION LOGICIELLE POUR IMAGERIE DYNAMIQUE AU POINT D'INTERVENTION

(30) Priority: 11.05.2017 US 201762504790 P
(43) Date of publication of application: 18.03.2020
(73) Proprietor: Carestream Health, Inc., Rochester, NY 14608 (US)
(72) Inventor: SCOTT, Richard T., Rochester NY 14608 (US); ROGERSON, Scott A., Rochester NY 14608 (US)
(74) Representative: Klang, Alexander H.
(86) International application number: PCT/US2018/032005
(87) International publication number: WO 2018/209045

(56) References cited:
- US-A1- 2011 111 703
- US-A1- 2011 170 669
- US-A1- 2015 106 645

## Description

### BACKGROUND OF THE INVENTION

The subject matter disclosed herein relates to radiographic image capture systems and methods that synchronize examination operations as between a digital radiographic (DR) detector and an x-ray generator, or x-ray source.

Fluoroscopy and tomosynthesis applications require both high-frame rate data capture and synchronization between a DR detector and an x-ray source. The synchronization allows the x-ray source to fire while the detector is integrating (integration window). Currently, it is desired to control timing operations of the DR detector and the x-ray source wirelessly for use in bedside applications. In some applications, wireless radiographic image capture occurs by triggering the detector to start integrating with timing uncertainty between the x-ray source's exposure period and the detector's integration period. The uncertainty is caused by uncontrollable elements, such as delays between the detector and control system as well as WiFi transmission delays. To compensate, the integration window is preset to a longer time window than the x-ray pulse width. However, a large integration window reduces the maximum frame rate, as measured in captured radiographic images (frames) per second (fps), and can limit the functionality of fluoroscopy and tomosynthesis applications.

With bedside dynamic imaging applications, such as fluoroscopy and tomosynthesis, it is important to keep the DR detector's integration window small while ensuring that the x-ray source is fired only during the integration window. It is desirable to synchronize the detector and x-ray source such that the x-ray source's period-ray pulse always falls within the DR detector's integration window. Further, it is desirable to minimize the latency between the user initiating the acquisition, i.e., the radiographic examination start, and the x-ray source firing.

The synchronization method is desired to have the following characteristics. A minimal timing error between the DR detector and the x-ray source is desired to minimize the DR detector's integration window; an ability to maintain synchronization over time in any environment, including temperature deviations and busy wireless networks; an ability to maintain synchronization over the entire examination period so that the user does not have to physically resynchronize at multiple times during the examination; and an ability to start, pause, and resume captures with minimal latency to avoid unintended additional exposures. It is desirable to maintain synchronization over the course of a day or between detector battery swaps. Fluoroscopy applications should have the ability to start acquiring images at any time and the user expects the images to be displayed with minimal latency.

Previous methods for timing operations between a DR detector and an x-ray source include a continuous beam detect method. Such a beam detect method is already programmed on the detector, where each image frame is analyzed for exposed image data and each frame with exposure data is transferred to a control system. This method can be used with a continuous exposure only. In a beam detect phase lock method, and using the beam detection method already programmed on the detector, the rising edge of the initial beam is detected and the subsequent frame capture timing is adjusted such that the x-ray-on time occurs during the integration window. Beam detection continues to occur with all image frames and captured image frames are sent to a control system if they contain exposure data. In a method of synchronized clocks with tethered synchronization, using a hardware signal, the DR detector and x-ray source are synchronized over the course of 1 to 2 seconds. Upon removal of the tether (cable), the synchronization is maintained for a finite amount of time. This method could be used with a schedule or with beam detect to export exposed frames.

The discussion above is merely provided for general background information and is not intended to be used as an aid in determining the scope of the claimed subject matter.

Reference is made to US 2011 / 111 703 A1 describing a synchronization method for a wireless link between a base station and a mobile cassette of an X-ray system. The base station includes an X-ray generator and the cassette includes an X-ray detector. The method includes steps for the exchange of messages between the base station and the cassette, the exchanges initiating timers in the base station and the cassette, a radiation sequence and an image sequence being executed at the end of the respective timers.

US 2015 / 106 645 A1 describes a method for synchronizing a plurality of components that are networked via a plurality of high speed switches, the method including frequency-locking to a master clock component and synchronizing to a master counter, driven by the master clock so that the frequency-locked component clocks drive the component counters in synchrony with the master counter.

### BRIEF DESCRIPTION OF THE INVENTION

In accordance with the present invention, a method of capturing radiographic images of a subject as set forth in Claim 1 is provided. Further embodiments of the invention are inter alia disclosed in the dependent claims. In particular, methods of synchronizing the firing of an x-ray source are disclosed with a capture (integration) window of a DR detector. The firing may start after the integration window is opened and may stop before the integration window is closed, to avoid unproductive patient exposure to x-rays.

One method of capturing radiographic images is carried out by wirelessly synchronizing steps performed by first and second components of a radiographic imaging system. Digital messages are sent between the first and second components and the send and receive times are recorded so that a transmission delay time and clock drift rate may be determined. A schedule of first component steps and a schedule of second component steps are generated based on the send and receive times, the transmission delay time and the clock drift rate.

In one embodiment, the x-ray source and the detector are connected over a Wi-Fi network. The x-ray source and detector exchange time stamped packets, wherein the sender, either the x-ray source or the detector, records its transmit and receive times as well as reading the time recorded in the time stamped response packet. Based on calculations using the recorded times and the time stamps, the sender determines the responder's local time and/or local clock relative to the sender's local time and/or clock and constructs an examination schedule to be sent to, and used by, the responder. Thus, the image capture sequence proceeds according to a predetermined schedule.

In one embodiment, a method of capturing radiographic images of a subject using a radiographic imaging system is disclosed. The radiographic imaging system includes a first component and a second component that are to be operated synchronously. A schedule of steps is generated for the first component with reference to a clock for the first component. Similarly, a schedule of steps is generated for the second component with reference to a clock for the second component. The clocks are used to time a start or stop for each of the steps in the corresponding first or second schedule. The schedules are generated after sending a first digital message over a digital network from the first component to the second component and sending a second digital message over the digital network from the second component to the first component. A time tA1 of the first component clock defines when the first digital message was sent by the first component. A time tB 1 of the second component clock defines when the first digital message was received at the second component. A time tA2 of the first component clock determines when the second digital message was received by the first component. The schedules of steps are generated based on the defined times tA1, tB1, and tA2, so that the first component steps and the second component steps are performed in synchrony.

This brief description of the invention is intended only to provide a brief overview of subject matter disclosed herein according to one or more illustrative embodiments, and does not serve as a guide to interpreting the claims or to define or limit the scope of the invention, which is defined only by the appended claims. This brief description is provided to introduce an illustrative selection of concepts in a simplified form that are further described below in the detailed description. This brief description is not intended to identify key features or essential features of the claimed subject matter, nor is it intended to be used as an aid in determining the scope of the claimed subject matter. The claimed subject matter is not limited to implementations that solve any or all disadvantages noted in the background.

### BRIEF DESCRIPTION OF THE DRAWINGS

So that the manner in which the features of the invention can be understood, a detailed description of the invention may be had by reference to certain embodiments, some of which are illustrated in the accompanying drawings. It is to be noted, however, that the drawings illustrate only certain embodiments of this invention and are therefore not to be considered limiting of its scope, for the scope of the invention encompasses other equally effective embodiments. The drawings are not necessarily to scale, emphasis generally being placed upon illustrating the features of certain embodiments of the invention. In the drawings, like numerals are used to indicate like parts throughout the various views. Thus, for further understanding of the invention, reference can be made to the following detailed description, read in connection with the drawings in which:
FIG. 1 is a schematic perspective view of an exemplary radiographic imaging system;
FIG. 2 is a schematic diagram of a photosensor array in a digital radiographic (DR) detector;
FIG. 3 is a perspective diagram of an exemplary DR detector;
FIG. 4 is a cross section diagram of an exemplary DR detector;
FIG. 5 is a flow diagram of an exemplary timing calculation to determine ASYM;
FIG. 6 is a flow diagram of an exemplary timing calculation to determine SYM;
FIG. 7 is a flow diagram of an exemplary timing calculation to determine a drift rate (DR) and to generate a schedule of steps for first and second components of the radiographic imaging system;
FIG. 8 is a flow diagram to generate a schedule of steps for first and second components of the radiographic imaging system without using a drift rate; and
FIG. 9 is a flow diagram to generate a schedule of steps for first and second components of the radiographic imaging system without using ASYM or a drift rate.

### DETAILED DESCRIPTION OF THE INVENTION

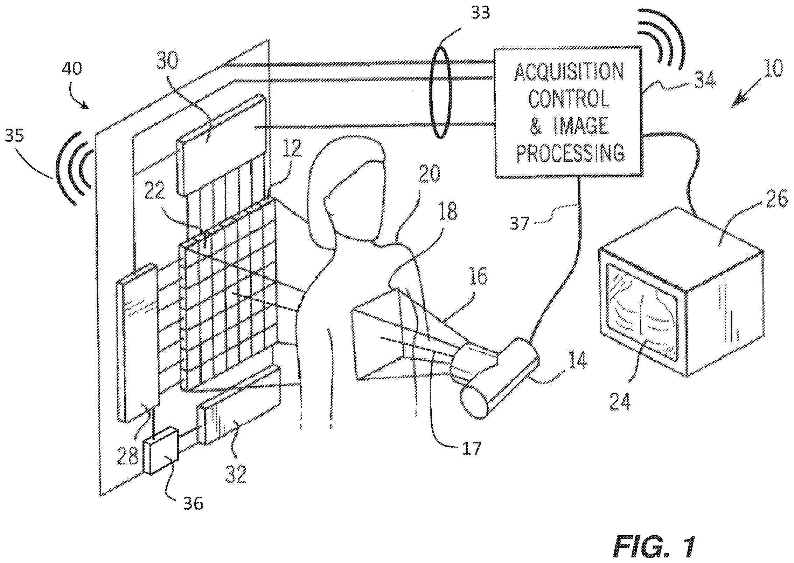

FIG. 1 is a perspective view of a digital radiographic (DR) imaging system 10 that may include a generally curved or planar DR detector 40 (shown in a planar embodiment and without a housing for clarity of description), an x-ray source 14 configured to generate radiographic energy (x-ray radiation), and a digital monitor, or electronic display, 26 configured to display images captured by the DR detector 40, according to one embodiment. The DR detector 40 may include a two dimensional array 12 of detector cells 22 (photosensors), arranged in electronically addressable rows and columns. The DR detector 40 may be positioned to receive x-rays 16 passing through a subject 20 during a radiographic energy exposure, or radiographic energy pulse, emitted by the x-ray source 14. As shown in FIG. 1, the radiographic imaging system 10 may use an x-ray source 14 that emits collimated x-rays 16, e.g. an x-ray beam, selectively aimed at and passing through a preselected region 18 of the subject 20. The x-ray beam 16 may be attenuated by varying degrees along its plurality of rays according to the internal structure of the subject 20, which attenuated rays are detected by the array 12 of photosensitive detector cells 22. The curved or planar DR detector 40 is positioned, as much as possible, in a perpendicular relation to a substantially central ray 17 of the plurality of rays 16 emitted by the x-ray source 14. In a curved array embodiment, the x-ray source 14 may be centrally positioned such that a larger percentage, or all, of the photosensitive detector cells are positioned perpendicular to incoming x-rays from the centrally positioned x-ray source 14. The array 12 of individual photosensitive cells (pixels) 22 may be electronically addressed (scanned) by their position according to column and row. As used herein, the terms "column" and "row" refer to the vertical and horizontal arrangement of the photosensor cells 22 and, for clarity of description, it will be assumed that the rows extend horizontally and the columns extend vertically. However, the orientation of the columns and rows is arbitrary and does not limit the scope of any embodiments disclosed herein. Furthermore, the term "subject" may be illustrated as a human patient in the description of FIG. 1, however, a subject of a DR imaging system, as the term is used herein, may be a human, an animal, an inanimate object, or a portion thereof.

In one exemplary embodiment, the rows of photosensitive cells 22 may be scanned one or more at a time by electronic scanning circuit 28 so that the exposure data from the array 12 may be transmitted to electronic read-out circuit 30. Each photosensitive cell 22 may independently store a charge proportional to an intensity, or energy level, of the attenuated radiographic radiation, or x-rays, received and absorbed in the cell. Thus, each photosensitive cell, when read-out, provides information defining a pixel of a radiographic image 24, e.g. a brightness level or an amount of energy absorbed by the pixel, that may be digitally decoded by image processing electronics 34 and transmitted to be displayed by the digital monitor 26 for viewing by a user. An electronic bias circuit 32 is electrically connected to the two-dimensional detector array 12 to provide a bias voltage to each of the photosensitive cells 22.

Each of the bias circuit 32, the scanning circuit 28, and the read-out circuit 30, may communicate with an acquisition control and image processing unit 34 over a connected cable 33 (wired), or the DR detector 40 and the acquisition control and image processing unit 34 may each be equipped with a wireless transmitter and receiver. Communication between the detector 40 and acquisition control and image processing unit 34 may be used by the acquisition control and image processing unit 34 to transmit commands to the detector 40 to begin capturing image data (begin integration). Wired or wireless communication 35 may be used by the detector 40 to transmit captured radiographic image data to the acquisition control and image processing unit 34, and for other communications as described herein. The acquisition control and image processing unit 34 may include a processor and electronic memory (not shown) to control operations of the DR detector 40 as described herein, including control of circuits 28, 30, and 32, for example, by use of programmed instructions, and to store and process image data.

The acquisition control and image processing unit 34 may also include a clock to be used for controlling activation of the x-ray source 14 during a radiographic examination sequence, such as an examination exposure start time and stop time according to a stored programmed schedule that makes reference to a clock time for synchronization purposes. The acquisition control and image processing unit 34 may also be connected to x-ray source 14 by cable or wire 37 used for controlling firing times for the x-ray source 14 and for controlling an x-ray tube electric current magnitude, and thus the fluence of x-rays in x-ray beam 16, and/or the x-ray tube voltage, and thus the energy level of the x-rays in x-ray beam 16. The acquisition control and image processing unit 34 may include input/output devices (not shown) such as a display monitor, keyboard and/or mouse, for communicating status and other information to a user and for receiving requests, commands, and instructions from the user for controlling operations, as described herein, of the detector 40 and x-ray source 14. For example, a user may use an input/output device to start, pause, and/or resume a fluoroscopic examination

A portion or all of the acquisition control and image processing unit 34 functions may reside in the detector 40, and be controlled by, an on-board processing system 36 which may include a processor and electronic memory to control operations of the DR detector 40 as described herein, including control of circuits 28, 30, and 32, by use of programmed instructions, and to store and process image data similar to the functions of standalone acquisition control and image processing system 34. The image processing system 36 may also include a clock to be used for timing integration start and stop times during a radiographic examination sequence according to a stored programmed schedule that makes reference to a DR detector clock time for synchronization purposes. The image processing system 36 may perform image acquisition and image disposition functions as described herein, such as controlling activation of the x-ray source 14 during a radiographic exposure, such as an examination exposure start time according to a programmed schedule generated by the image processing system 36. The image processing system 36 may control image transmission and image processing and image correction on board the detector 40 based on instructions or other commands transmitted from the acquisition control and image processing unit 34, and transmit corrected digital image data therefrom. Alternatively, acquisition control and image processing unit 34 may receive raw image data from the detector 40 and process the image data and store it, or it may store raw unprocessed image data in local memory, or in remotely accessible memory.

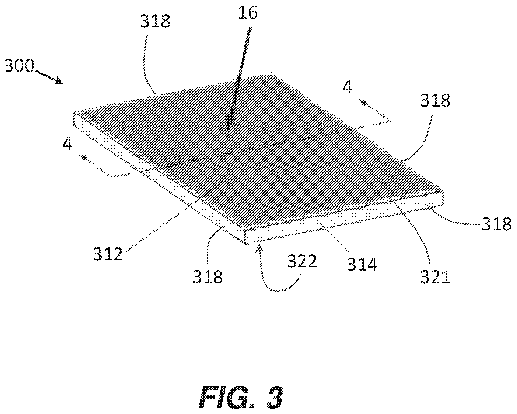

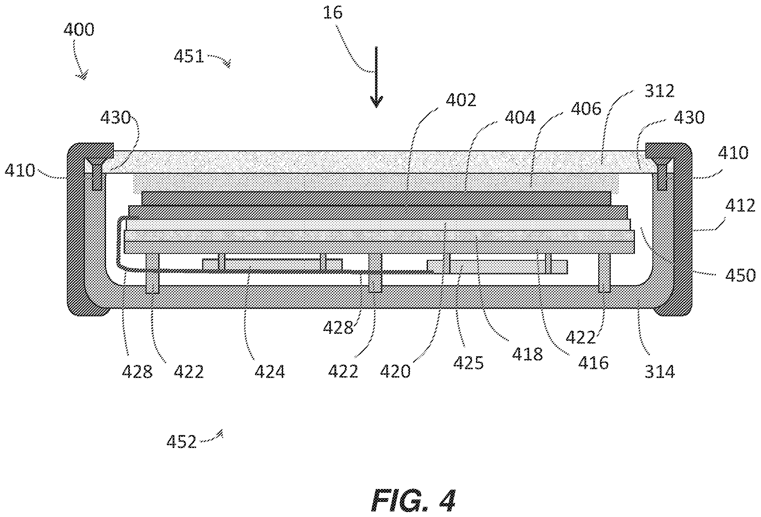

With regard to a direct detection embodiment of DR detector 40, the photosensitive cells 22 may each include a sensing element sensitive to x-rays, i.e. it absorbs x-rays and generates an amount of charge carriers in proportion to a magnitude of the absorbed x-ray energy. A switching element may be configured to be selectively activated to read out the charge level of a corresponding x-ray sensing element. With regard to an indirect detection embodiment of DR detector 40, photosensitive cells 22 may each include a sensing element sensitive to light rays in the visible spectrum, i.e. it absorbs light rays and generates an amount of charge carriers in proportion to a magnitude of the absorbed light energy, and a switching element that is selectively activated to read the charge level of the corresponding sensing element. A scintillator, or wavelength converter, may be disposed over the light sensitive sensing elements to convert incident x-ray radiographic energy to visible light energy. Thus, in the embodiments disclosed herein, it should be noted that the DR detector 40 (or DR detector 300 in FIG. 3 or DR detector 400 in FIG. 4) may include an indirect or direct type of DR detector.

Examples of sensing elements used in sensing array 12 include various types of photoelectric conversion devices (e.g., photosensors) such as photodiodes (P-N or PIN diodes), photo-capacitors (MIS), photo-transistors or photoconductors. Examples of switching elements used for signal read-out include a-Si TFTs, oxide TFTs, MOS transistors, bipolar transistors and other p-n junction components.

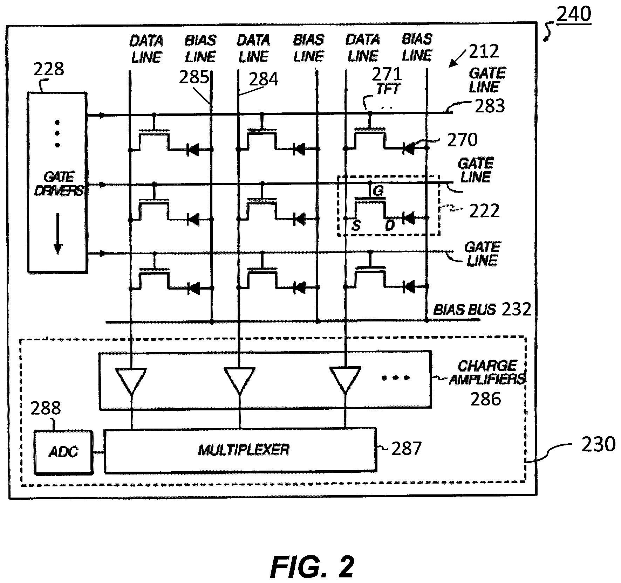

FIG. 2 is a schematic diagram 240 of a portion of a two-dimensional array 12 for a DR detector 40. The array of photosensor cells 212, whose operation may be consistent with the photosensor array 12 described above, may include a number of hydrogenated amorphous silicon (a-Si:H) n-i-p photodiodes 270 and thin film transistors (TFTs) 271 formed as field effect transistors (FETs) each having gate (G), source (S), and drain (D) terminals. In embodiments of DR detector 40 disclosed herein, such as a multilayer DR detector (400 of FIG. 4), the two-dimensional array of photosensor cells 12 may be formed in a device layer that abuts adjacent layers of the DR detector structure, which adjacent layers may include a rigid glass layer or a flexible polyimide layer or a layer including carbon fiber without any adjacent rigid layers. A plurality of gate driver circuits 228 may be electrically connected to a plurality of gate lines 283 which control a voltage applied to the gates of TFTs 271, a plurality of readout circuits 230 may be electrically connected to data lines 284, and a plurality of bias lines 285 may be electrically connected to a bias line bus or a variable bias reference voltage line 232 which controls a voltage applied to the photodiodes 270. Charge amplifiers 286 may be electrically connected to the data lines 284 to receive signals therefrom. Outputs from the charge amplifiers 286 may be electrically connected to a multiplexer 287, such as an analog multiplexer, then to an analog-to-digital converter (ADC) 288, or they may be directly connected to the ADC, to stream out the digital radiographic image data at desired rates. In one embodiment, the schematic diagram of FIG. 2 may represent a portion of a DR detector 40 such as an a-Si:H based indirect flat panel, curved panel, or flexible panel imager.

Incident x-rays, or x-ray photons, 16 are converted to optical photons, or light rays, by a scintillator, which light rays are subsequently converted to electron-hole pairs, or charges, upon impacting the a-Si:H n-i-p photodiodes 270. In one embodiment, an exemplary detector cell 222, which may be equivalently referred to herein as a pixel, may include a photodiode 270 having its anode electrically connected to a bias line 285 and its cathode electrically connected to the drain (D) of TFT 271. The bias reference voltage line 232 can control a bias voltage of the photodiodes 270 at each of the detector cells 222. The charge capacity of each of the photodiodes 270 is a function of its bias voltage and its capacitance. In general, a reverse bias voltage, e.g. a negative voltage, may be applied to the bias lines 285 to create an electric field (and hence a depletion region) across the pn junction of each of the photodiodes 270 to enhance its collection efficiency for the charges generated by incident light rays. The image signal represented by the array of photosensor cells 212 may be integrated by the photodiodes while their associated TFTs 271 are held in a non-conducting (off) state, for example, by maintaining the gate lines 283 at a negative voltage via the gate driver circuits 228. The photosensor cell array 212 may be read out by sequentially switching rows of the TFTs 271 to a conducting (on) state by means of the gate driver circuits 228. When a row of the pixels 22 is switched to a conducting state, for example by applying a positive voltage to the corresponding gate line 283, collected charge from the photodiode in those pixels may be transferred along data lines 284 and integrated by the external charge amplifier circuits 286. The row may then be switched back to a non-conducting state, and the process is repeated for each row until the entire array of photosensor cells 212 has been read out. The integrated signal outputs are transferred from the external charge amplifiers 286 to an analog-to-digital converter (ADC) 288 using a parallel-to-serial converter, such as multiplexer 287, which together comprise read-out circuit 230.

This digital image information may be subsequently processed by image processing system 34 to yield a digital image which may then be digitally stored and immediately displayed on monitor 26, or it may be displayed at a later time by accessing the digital electronic memory containing the stored image. The flat panel DR detector 40 having an imaging array as described with reference to FIG. 2 is capable of both single-shot (e.g., static, radiographic) and continuous (e.g., fluoroscopic) image acquisition.

FIG. 3 shows a perspective view of an exemplary prior art generally rectangular, planar, portable wireless DR detector 300 according to an embodiment of DR detector 40 disclosed herein. The DR detector 300 may include a flexible substrate to allow the DR detector to capture radiographic images in a curved orientation. The flexible substrate may be fabricated in a permanent curved orientation, or it may remain flexible throughout its life to provide an adjustable curvature in two or three dimensions, as desired. The DR detector 300 may include a similarly flexible housing portion 314 that surrounds a multilayer structure comprising a flexible photosensor array portion 22 of the DR detector 300. The housing portion 314 of the DR detector 300 may include a continuous, rigid or flexible, x-ray opaque material or, as used synonymously herein a radio-opaque material, surrounding an interior volume of the DR detector 300. The housing portion 314 may include four flexible edges 318, extending between the top side 321 and the bottom side 322, and arranged substantially orthogonally in relation to the top and bottom sides 321, 322. The bottom side 322 may be continuous with the four edges and disposed opposite the top side 321 of the DR detector 300. The top side 321 comprises a top cover 312 attached to the housing portion 314 which, together with the housing portion 314, substantially encloses the multilayer structure in the interior volume of the DR detector 300. The top cover 312 may be attached to the housing 314 to form a seal therebetween, and be made of a material that passes x-rays 16 without significant attenuation thereof, i.e., an x-ray transmissive material or, as used synonymously herein, a radiolucent material, such as a carbon fiber plastic, polymeric, or other plastic based material.

With reference to FIG. 4, there is illustrated in schematic form an exemplary cross-section view along section 4-4 of the exemplary embodiment of the DR detector 300 (FIG. 3). For spatial reference purposes, one major surface of the DR detector 400 may be referred to as the top side 451 and a second major surface may be referred to as the bottom side 452, as used herein. The multilayer structure may be disposed within the interior volume 450 enclosed by the housing 314 and top cover 312 and may include a flexible curved or planar scintillator layer 404 over a curved or planar the two-dimensional imaging sensor array 12 shown schematically as the device layer 402. The scintillator layer 404 may be directly under (e.g., directly connected to) the substantially planar top cover 312, and the imaging array 402 may be directly under the scintillator 404. Alternatively, a flexible layer 406 may be positioned between the scintillator layer 404 and the top cover 312 as part of the multilayer structure to allow adjustable curvature of the multilayer structure and/or to provide shock absorption. The flexible layer 406 may be selected to provide an amount of flexible support for both the top cover 312 and the scintillator 404, and may comprise a foam rubber type of material. The layers just described comprising the multilayer structure each may generally be formed in a rectangular shape and defined by edges arranged orthogonally and disposed in parallel with an interior side of the edges 318 of the housing 314, as described in reference to FIG. 3.

A substrate layer 420 may be disposed under the imaging array 402, such as a rigid glass layer, in one embodiment, or flexible substrate comprising polyimide or carbon fiber upon which the array of photosensors 402 may be formed to allow adjustable curvature of the array, and may comprise another layer of the multilayer structure. Under the substrate layer 420 a radio-opaque shield layer 418 may be used as an x-ray blocking layer to help prevent scattering of x-rays passing through the substrate layer 420 as well as to block x-rays reflected from other surfaces in the interior volume 450. Readout electronics, including the scanning circuit 28, the read-out circuit 30, the bias circuit 32, and processing system 36 (all of FIG. 1) may be formed adjacent the imaging array 402 or, as shown, may be disposed below frame support member 416 in the form of integrated circuits (ICs) electrically connected to printed circuit boards 424, 425. The imaging array 402 may be electrically connected to the readout electronics 424 (ICs) over a flexible connector 428 which may comprise a plurality of flexible, sealed conductors known as chip-on-film (COF) connectors.

X-ray flux may pass through the radiolucent top panel cover 312, in the direction represented by an exemplary x-ray beam 16, and impinge upon scintillator 404 where stimulation by the high-energy x-rays 16, or photons, causes the scintillator 404 to emit lower energy photons as visible light rays which are then received in the photosensors of imaging array 402. The frame support member 416 may connect the multilayer structure to the housing 314 and may further operate as a shock absorber by disposing elastic pads (not shown) between the frame support beams 422 and the housing 314. Fasteners 410 may be used to attach the top cover 312 to the housing 314 and create a seal therebetween in the region 430 where they come into contact. In one embodiment, an external bumper 412 may be attached along the edges 318 of the DR detector 400 to provide additional shock-absorption.

In a method of synchronizing clocks corresponding to components of the radiographic imaging system using communications over a WiFi channel, two exemplary clocks, clock A and clock B, or simply A and B, are referenced herein which may define clocks that control timing of a schedule of operations corresponding to the x-ray source and the DR detector. Random communication time delays from A to B and from B to A introduce potential timing errors. A to B and B to A delays may not be equal or consistent over time and are characterized by typical randomness and occasional extreme outlier randomness. Also, the clocks drift relative to each other over time. A and B clocks may reference an event schedule that specifies operation start and stop times that occur at a future time. As described herein, clock A will manage all the corrections and adjustments in order to align its event schedule with clock B according to its offset and drift calculations and corrections.

An example schedule of steps for A and B may be arranged as shown in the following table, whereby the schedule of steps and corresponding times for execution according to local times for clocks A and B are shown in respective columns and are determined, in general, by a formula such as B schedule = A schedule + Offset, wherein the Offset is calculated as described herein. The numerical entries in the table refer to a local running clock time (for clock A and for clock B) which may be measured by any desired units of time and which, for example, may refer to milliseconds. It will be understood that any time reference may be used for clocks A and B, such as a time-of-day clock, a world clock, an agreed time start at a defined t₀, or some other suitable reference.

| A STEP | B STEP | CLOCK A | CLOCK B |
|---|---|---|---|
| - | Start Integ | | 17104 |
| Start Exposure | - | 15560 | |
| End Exposure | - | 15563 | |
| - | End Integ | | 17129 |
| - | Start Integ | | 17141 |
| Start Exposure | - | 15593 | |
| End Exposure | - | 15596 | |
| - | End Integ | | 17166 |

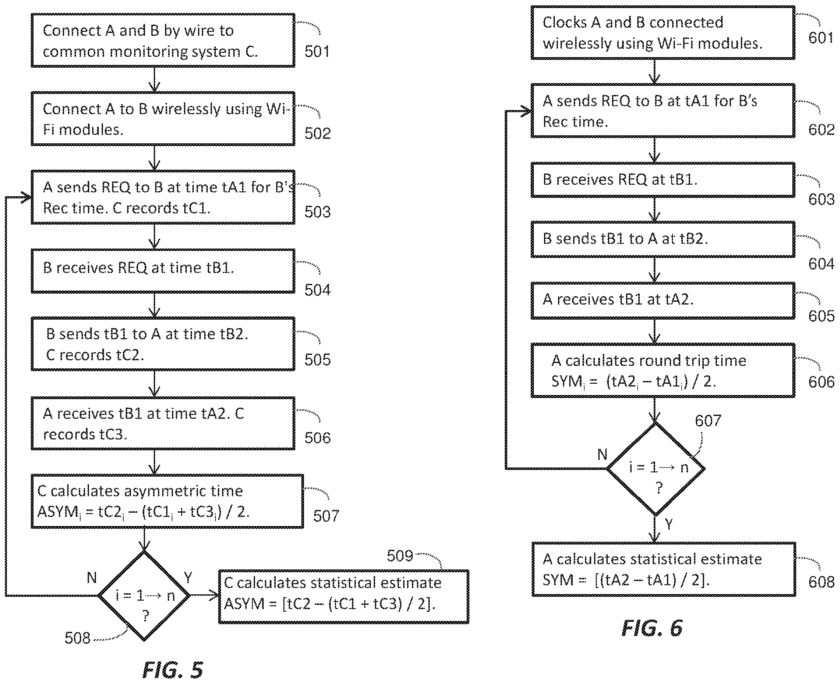

FIG. 5 is a flow chart of a method to evaluate a variable ASYM which represents a difference in transmission delay times as between wireless transmission times from B to A and from A to B. In a first step 501, A and B are connected by cable or wire to a common monitoring system C which may include an oscilloscope for precise simultaneous time measurement of events occurring at A and B. In a second step 502, A and B are connected to each other over a WiFi network to establish wireless communication therebetween. At step 503, A sends a request (REQ), at a time tA1, to B for its receive time. A may or may not record its local time tA1, however, C records the send time tC1 as measured by C. At step 504, B receives the request at time tB1, and may or may not record its local time tB1. At step 505, B sends tB1 to A at time tB2, and may or may not record its local time tB2, however, C records the send time tC2 as measured by C. At step 506, A receives tB1 at local time tA2. A may or may not record its local time tA2, however, C records the receive time tC3 as measured by C. At step 507, C calculates ASYMᵢ using the formula ASYMᵢ = tC2ᵢ - (tC1ᵢ + tC3ᵢ) / 2. At step 508, A determines whether a desired number n of sample measurements *i* of times tC1, tC2, and tC3 has been performed. If not, the method returns to step 503. If the desired number *n* of sample measurements *i* has been performed as determined at step 508, then, at step 509, C calculates ASYM using the formula ASYM = [tC2 - (tC1 + tC3) / 2], which is stored by A for use in the field, as described herein. ASYM represents a statistical estimate of the individual calculations of ASYM_{1→n} = tC2_{1→n} - (tC1 _{1→n} + tC3_{1→n}) / 2. It will be noted that the statistical estimate may include an average, a median, some other measure of central tendency, an outlier rejection method, an order statistic, a weighted moving average whether linear or exponential, a regression, and other desired statistical measures, or combinations thereof. If *n* is selected to be equal to one (1), then a single calculation is used to evaluate ASYM.

The sequence of steps 503 - 507 may be performed several times to obtain several samples of measured times tC1, tC2, and tC3 and/or reported send and receive times tA1, tA2, tB1, and tB2. The sequence of steps 503 - 507 may be performed one (1) time, five hundred (500) times, or they may be performed ten thousand (10,000) or one hundred thousand (100,000) times, or they may be performed one million (1,000,000) times, or any other desired number of times. As described herein, the statistical measures may be used to determine a communication delay between the components using clocks A and B and/or a relative time difference as between clocks A and B. The measured delays and relative time differences may be used to generate separate schedules for separate components of the radiographic imaging system that uses clocks A and B, respectively, to time the steps performed by the separate components according to their respective schedules so that the steps performed occur in a desired timed sequence, i.e., in synchrony.

FIG. 6 is a flow chart of a method to evaluate a variable SYM which represents a symmetric transmission delay time as between wireless transmission times from B to A and from A to B. In a first step 601, A and B are connected to each other wirelessly over a WiFi communication channel to establish wireless communication therebetween. At step 602, A sends a request (REQ), at A time tA1, to B for its receive time and records time tA1. At step 603, B receives the request at time tB1 and records tB1. At step 604, B sends tB1 to A at time tB2 and may or may not record its local time tB2. At step 605, A receives tB1 at local time tA2 and records its local time tA2. At step 606, A calculates a round trip time SYMᵢ using the formula SYMᵢ = (tA2ᵢ - tA1ᵢ) / 2. At step 607, A determines whether a desired number *n* of sample measurements *i* of times tA1 and tA2 has been performed. If not, the method returns to step 602. If the desired number *n* of sample measurements *i* has been performed as determined at step 607, then, at step 608, A calculates SYM using the formula SYM = [(tA2 - tA1) / 2], which is stored by A, as described herein. SYM represents a statistical estimate of the individual calculations of SYM_{1→n} = (tA2_{1→n} - tA1_{1→n}) / 2. It will be noted that the statistical estimate may include an average, a median, some other measure of central tendency, an outlier rejection method, an order statistic, a weighted moving average whether linear or exponential, a regression, and other desired measures, or combinations thereof. If *n* is selected to be equal to one (1), then a single calculation is used to evaluate SYM.

The sequence of steps 602 - 606 may be performed several times to obtain several samples of measured times tA1 and tA2 and/or reported send and receive times tB1 and tB2. The sequence of steps 602 - 606 may be performed one (1) time, five hundred (500) times, or they may be performed ten thousand (10,000) or one hundred thousand (100,000) times, or they may be performed one million (1,000,000) times, or any other desired number of times. As described herein, the statistical measures may be used to determine a symmetric communication delay between the components using clocks A and B and/or a relative time difference as between clocks A and B. The measured delays and relative time differences may be used to generate separate schedules for separate components of the radiographic imaging system that uses clocks A and B, respectively, to time the steps performed by the separate components according to their respective schedules so that the steps performed occur in a desired timed sequence, i.e., in synchrony.

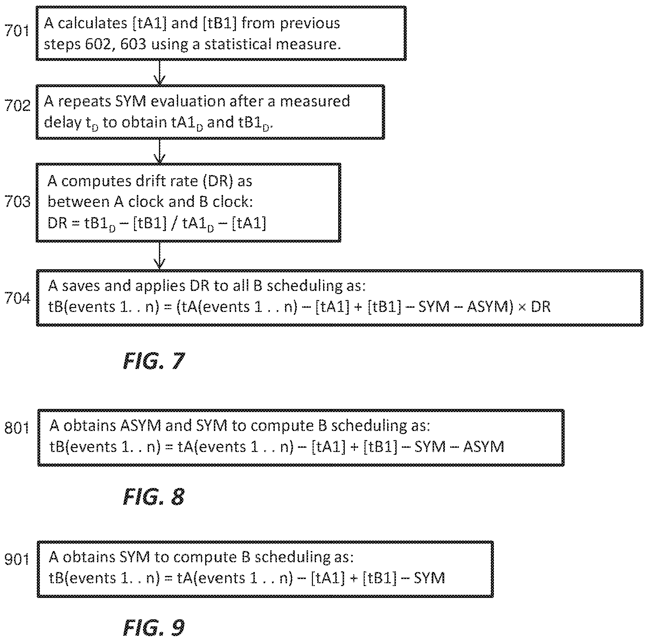

FIG. 7 is a flow chart of a method to evaluate the variable DR which represents a measure of a rate of change, or drift, of a relative time difference as between clocks A and B. The flow chart of FIG. 7 also shows a step of generating a schedule of steps, such as the exemplary schedule of steps shown in the table above, using the determined value of DR. In one embodiment, the flow chart of FIG. 7 may be initiated after the method of determining ASYM, as shown in the flow chart of FIG. 5, and SYM, as shown in the flow chart of FIG. 6, are performed. At step 701, A retrieves the times tA1_{1→n} and tB1_{1→n} obtained during the method of evaluating SYM (FIG. 6) at steps 602, 603, and, selecting any one of several statistical estimates described herein, determines representative values [tA1] and [tB1], respectively. At step 702, the SYM evaluation of FIG. 6 is repeated and new values tA1_{D} and tB1_{D} are determined at steps 602, 603, respectively, after a delay t_{D} from the immediately previous measurements thereof. The repeated SYM evaluation may be performed with *n* = 1 in which case the new values tA1_{D} and tB1_{D} are determined directly. The repeated SYM evaluation may be performed with n = x, where x is some other integer greater than one (1), in which case any one of the several statistical estimates described herein may be used to determine the new values tA1_{D} and tB1_{D}. At step 703, the drift rate (DR) is computed according to the formula DR = tB1_{D} - [tB1] / tA1_{D} - [tA1].

At step 704, a schedule of steps for A and B may be computed using the formula tB(events 1.. n) = (tA(events 1 .. n) - [tA1] + [tB1] - SYM - ASYM) × DR, wherein tB(events 1, 2, 3, 4) may represent a schedule of steps transmitted to a processor 36 of a DR detector 40 to be performed thereby. In one embodiment, the schedule of steps may be determined by the processor 36 of DR detector 40 if the DR detector is the component controlled by clock A which may be implemented as an on-board clock for the DR detector 40. In that event, the column labels "A step" and "B step" would be interchanged. (events 1, 2, 3, 4) may represent 1 start integration, 2 end integration, 3 start integration, 4 end integration, as shown in the column "B step" in the table above. The first integration start begins when B's clock reaches 17104, and so on, according to the schedule of steps. Similarly, tA(events 1, 2, 3, 4) may represent a schedule of steps transmitted to a processor of the acquisition control and image processing unit 34 to be performed thereby via control over x-ray source 14. In the embodiment as shown in the table above, the schedule of steps is determined by the processor of the acquisition control and image processing unit 34 which includes clock A for timing operation of the x-ray source 14. (events 1, 2, 3, 4) may represent 1 start exposure, 2 end exposure, 3 start exposure, 4 end exposure, as shown in the column "A step" in the table above. The first exposure starts when A's clock reaches 15560, and so on, according to the schedule of steps.

A may be programmed to use the determined DR at preselected intervals to compensate its schedule of steps to maintain synchronous operation with B. This compensation may include A adding or subtracting milliseconds on the fly to the times for its scheduled steps yet to be performed, e.g., the times in the column labeled "Clock A" in the table above. The length of time to be used for the preselected compensation intervals will depend on a tolerance level desired to be designed into the drift compensation and on the actual measured DR. Typically, a greater DR or a tighter desired tolerance level will require more frequent compensation adjustments to A's schedule of steps yet to be performed. In general, an amount of time compensation required for A may be determined by (±DR × selected time interval).

In one embodiment, the determined DR may be used to adjust the schedule of steps for A and B after a user pauses or stops (interrupts) performance of the schedule of steps, such as in the middle of a fluoroscopic examination, for example. In one embodiment, A's clock may be monitored to determine an amount of time that has elapsed since the user paused the examination until the user requests resumption of the examination's schedule of steps. Using (±DR × elapsed time), A may then quickly update each of the times for the remaining schedule of steps, or reschedule the entire schedule of steps, and send the update or reschedule of steps to B for resuming or restarting the fluoroscopic examination, without requiring that A reevaluate SYM, ASYM, or DR, as described herein.

FIG. 8 is a flow chart of a method to generate a schedule of steps for A and B as in the table above, and as explained above in relation to FIG. 7, except that the method does not require evaluation of DR. In one embodiment, the flow chart of FIG. 8 may be initiated after the method of determining ASYM, as shown in the flow chart of FIG. 5, and SYM, as shown in the flow chart of FIG. 6, are performed. At step 801, a schedule of steps for A and B may be computed using the formula tB(events 1.. n) = tA(events 1 .. n) - [tA1] + [tB1] - SYM - ASYM.

FIG. 9 is a flow chart of a method to generate a schedule of steps for A and B as in the table above, and as explained above in relation to FIG. 7, except that the method does not require evaluation of DR or ASYM. In one embodiment, the flow chart of FIG. 9 may be initiated after the method of determining SYM, as shown in the flow chart of FIG. 6, is performed. At step 901, a schedule of steps for A and B may be computed using the formula tB(events 1.. n) = tA(events 1 .. n) - [tA1] + [tB1] - SYM.

As will be appreciated by one skilled in the art, aspects of the present invention may be embodied as a system, method, or computer program product. Accordingly, aspects of the present invention may take the form of an entirely hardware embodiment, an entirely software embodiment (including firmware, resident software, micro-code, etc.), or an embodiment combining software and hardware aspects that may all generally be referred to herein as a "service," "circuit," "circuitry," "module," and/or "system." Furthermore, aspects of the present invention may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

Program code and/or executable instructions embodied on a computer readable medium may be transmitted using any appropriate medium, including but not limited to wireless, wireline, optical fiber cable, RF, etc., or any suitable combination of the foregoing. Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer (device), partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described herein with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks. The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

## Claims

1. A method of capturing radiographic images of a subject using a radiographic imaging system, the radiographic imaging system including a first component and a second component, the method comprising:
generating a schedule of first component steps (704; 804; 904) to be performed by the first component, the schedule of first component steps (704; 804; 904) each corresponding to a first component clock time referenced by the first component to start and stop each of the first component steps (704; 804; 904); and
generating a schedule of second component steps (704; 804; 904) to be performed by the second component, the schedule of second component steps (704; 804; 904) each corresponding to a second component clock time referenced by the second component to start and stop each of the second component steps (704; 804; 904),
wherein the steps of generating the schedule of first component steps (704; 804; 904) and generating the schedule of second component steps (704; 804; 904) comprise:
sending (503; 602) a first digital message over a digital network from the first component to the second component and sending (505; 604) a second digital message over the digital network from the second component to the first component;
determining a time tA1 of the first component clock when the first digital message was sent by the first component;
determining a time tB1 of the second component clock when the first digital message was received at the second component;
determining a time tA2 of the first component clock when the second digital message was received by the first component; and
generating the schedule of first component steps (704; 804; 904) and generating the schedule of second component steps (704; 804; 904) based on the determined times tA1, tB1, arid tA2, such that the first component steps (704; 804; 904) and the second component steps (704; 804; 904) are performed in synchrony.

2. The method of claim 1, further comprising statistically determining a communication delay between the first component clock and the second component clock based on repeating the steps of sending (503; 602) the first digital message and sending (505; 604) the second digital message and determining a plurality of the times tA1_{i→n}, tB1_{i→n}, and tA2_{i→n}.

3. The method of claim 2, further comprising statistically determining a relative clock time difference as between the first component clock time and the second component clock time based on repeating the steps of sending (503; 602) the first digital message and sending (505; 604) the second digital message and determining the plurality of the times tA1_{i→n}, tB1_{i→n}, and tA2_{i→n}.

4. The method of claim 1, further comprising repeating the steps of sending (503; 602) the first digital message and sending (505; 604) the second digital message and determining a plurality of times tB2_{i→n} of the second component clock when the second digital messages were sent by the second component.

5. The method of claim 4, wherein the step of generating (801) the schedule of first component steps (704; 804; 904), tA(events 1.. n), and generating (801) the schedule of second component steps (704; 804; 904), tB(events 1.. n), includes using the formula tB(events 1.. n) = tA(events 1 .. n) - tA1 + tB1 - ((tA2 - tA1) / 2) - (tB2 - (tA1 + tA2) / 2).

6. The method of claim 1, further comprising connecting the first component and the second component to a common monitoring system, wherein the common monitoring system performs the steps of:
determining a time tC1 of the first component clock when the first digital message was sent by the first component;
determining a time tC2 of the second component clock when the second digital message was sent by the second component; and
determining a time tC3 of the first component clock when the second digital message was received by the first component,
wherein the step of generating (801) the schedule of first component steps (704; 804; 904), tA(events 1.. n), and generating (801) the schedule of second component steps (704; 804; 904), tB(events 1.. n), includes using the formula tB(events 1.. n) = tA(events 1 .. n) - tA1 + tB1 - ((tA2 - tA1) / 2) - (tC2 - (tC1 + tC3) / 2).

7. The method of claim 4, further comprising determining a drift rate, DR, using the formula DR = (tB1ᵢ - tB1_{i→1}) / (tA1ᵢ - tA1_{i→1})

8. The method of claim 7, wherein the step of generating (704) the schedule of first component steps (704; 804; 904), tA(events 1.. n), and generating (704) the schedule of second component steps (704; 804; 904), tB(events 1.. n), includes using the formula tB(events 1.. n) = [tA(events 1 .. n) - tA1 + tB 1 - ((tA2 - tA1) / 2) - (tB2 - (tA1 + tA2) / 2)] × DR.

9. The method of claim 6, further comprising determining a drift rate, DR, using the formula DR = (tB1ᵢ - tB1_{i→1}) / (tA1ᵢ - tA1_{i→1})

10. The method of claim 9, wherein the step of generating (704) the schedule of first component steps (704; 804; 904), tA(events 1.. n), and generating (704) the schedule of second component steps (704; 804; 904), tB(events 1.. n), includes using the formula tB(events 1.. n) = [tA(events 1 .. n) - tA1 + tB1 - ((tA2 - tA1) / 2) - (tC2 - (tC1 + tC3) / 2)] × DR.

11. The method of claim 1, further comprising scheduling an integration start time and end time for a digital radiographic detector in the schedule of second component steps (704; 804; 904).

12. The method of claim 11, further comprising scheduling an exposure start time and end time for an x-ray source in the schedule of first component steps (704; 804; 904).

13. The method of claim 12, further comprising:
the radiographic imaging system performing the schedule of steps;
pausing performance of the schedule of steps;
resuming performance of the schedule of steps;
determining an elapsed time between the steps of pausing and resuming; and
rescheduling unperformed steps in the paused performance of the schedule of steps,
wherein the step of rescheduling is based on a drift rate between the first component clock time and the second component clock time, the drift rate being used to update the times in the rescheduled unperformed steps, and wherein the rescheduled unperformed steps are used in the step of resuming performance of the schedule of steps.

14. The method of claim 12, further comprising:
the radiographic imaging system performing the schedule of steps;
stopping performance of the schedule of steps;
restarting performance of the schedule of steps;
determining an elapsed time between the steps of stopping and restarting; and
rescheduling the steps in the schedule of steps,
wherein the step of rescheduling is based on a drift rate between the first component clock time and the second component clock time, the drift rate being used to reschedule the steps in the schedule of steps, and wherein the rescheduled steps are used in the step of restarting.

## Patentansprüche

1. Ein Verfahren zum Erfassen von radiographischen Bildern eines Subjekts unter Verwendung eines radiographischen Bildgebungssystems, wobei das radiographische Bildgebungssystem eine erste Komponente und eine zweite Komponente enthält, wobei das Verfahren Folgendes aufweist:
Erzeugen eines Ablauf- bzw. Zeitplans von Schritten (704; 804; 904) der ersten Komponente, die von der ersten Komponente auszuführen sind, wobei der Zeitplan der Schritte (704; 804; 904) der ersten Komponente jeweils einer Taktzeit der ersten Komponente entspricht, auf die sich die erste Komponente bezieht, um jeden der Schritte (704; 804; 904) der ersten Komponente zu starten und zu stoppen; und
Erzeugen eines Ablauf- bzw. Zeitplans von Schritten (704; 804; 904) der zweiten Komponente, die von der zweiten Komponente auszuführen sind, wobei der Zeitplan von Schritten (704; 804; 904) der zweiten Komponente jeweils einer Taktzeit der zweiten Komponente entspricht, auf die sich die zweite Komponente bezieht, um jeden der Schritte (704; 804; 904) der zweiten Komponente zu starten und zu stoppen,
wobei die Schritte des Erzeugens des Zeitplans von Schritten (704; 804; 904) der ersten Komponente und des Erzeugens des Zeitplans von Schritten (704; 804; 904) der zweiten Komponente Folgendes aufweisen: Senden (503; 602) einer ersten digitalen Nachricht über ein digitales Netzwerk von der ersten Komponente zu der zweiten Komponente und Senden (505; 604) einer zweiten digitalen Nachricht über das digitale Netzwerk von der zweiten Komponente zu der ersten Komponente;
Bestimmen eines Zeitpunkts tA1 der Taktzeit der ersten Komponente, wenn bzw. sobald die erste digitale Nachricht von der ersten Komponente gesendet wurde;
Bestimmen eines Zeitpunktes tB1 der Taktzeit der zweiten Komponente, wenn bzw. sobald die erste digitale Nachricht an der zweiten Komponente empfangen wurde;
Bestimmen eines Zeitpunkts tA2 der Taktzeit der ersten Komponente, wenn bzw. sobald die zweite digitale Nachricht von der ersten Komponente empfangen wurde; und
Erzeugen des Zeitplans der Schritte der ersten Komponente (704; 804; 904) und Erzeugen des Zeitplans der Schritte der zweiten Komponente (704; 804; 904) basierend auf den bestimmten Zeitpunkten tA1, tB1 und tA2, so dass die Schritte der ersten Komponente (704; 804; 904) und die Schritte der zweiten Komponente (704; 804; 904) synchron ausgeführt werden.

2. Das Verfahren nach Anspruch 1, das ferner Folgendes aufweist:
statistisches Bestimmen einer Kommunikationsverzögerung zwischen der Taktzeit der ersten Komponente und der Taktzeit der zweiten Komponente, und zwar basierend auf Wiederholen der Schritte des Sendens (503; 602) der ersten digitalen Nachricht und des Sendens (505; 604) der zweiten digitalen Nachricht und auf Bestimmen einer Vielzahl der Zeitpunkte tA1_{i→n}, tB1_{i→n} und tA2_{i→n}.

3. Das Verfahren nach Anspruch 2, das ferner Folgendes aufweist:
statistisches Bestimmen einer relativen Taktzeitdifferenz zwischen der Taktzeit der ersten Komponente und der Taktzeit der zweiten Komponente, und zwar basierend auf Wiederholen der Schritte des Sendens (503; 602) der ersten digitalen Nachricht und des Sendens (505; 604) der zweiten digitalen Nachricht und auf Bestimmen der Vielzahl der Zeitpunkte tA1_{i→n}, tB1_{i→n} und tA2_{i→n}.

4. Das Verfahren nach Anspruch 1, das ferner Folgendes aufweist:
Wiederholen der Schritte des Sendens (503; 602) der ersten digitalen Nachricht und des Sendens (505; 604) der zweiten digitalen Nachricht und Bestimmen einer Vielzahl von Zeitpunkten tB2_{i→n} der Taktzeit der zweiten Komponente, wenn bzw. sobald die zweiten digitalen Nachrichten von der zweiten Komponente gesendet wurden.

5. Das Verfahren nach Anspruch 4, wobei der Schritt des Erzeugens (801) des Zeitplans der Schritte der ersten Komponente (704; 804; 904), mit tA(Ereignisse 1 bis n), und des Erzeugens (801) des Zeitplans der Schritte der zweiten Komponente (704; 804; 904), mit tB(Ereignisse 1 bis n), Verwenden der Formel tB(Ereignisse 1bis n) = tA(Ereignisse 1 bis n) - tA1 + tB1 - ((tA2 - tA1) / 2) - (tB2 - (tA1 + tA2) / 2) aufweist.

6. Das Verfahren nach Anspruch 1, das ferner Folgendes aufweist:
Verbinden der ersten Komponente und der zweiten Komponente mit einem gemeinsamen Überwachungssystem, wobei das gemeinsame Überwachungssystem die folgenden Schritte durchführt:
Bestimmen eines Zeitpunkts tC1 der Taktzeit der ersten Komponente, wenn bzw. sobald die erste digitale Nachricht von der ersten Komponente gesendet wurde;
Bestimmen eines Zeitpunkts tC2 der Taktzeit der zweiten Komponente, wenn bzw. sobald die zweite digitale Nachricht von der zweiten Komponente gesendet wurde; und
Bestimmen eines Zeitpunkts tC3 der Taktzeit der ersten Komponente, wenn bzw. sobald die zweite digitale Nachricht von der ersten Komponente empfangen wurde,
wobei der Schritt des Erzeugens (801) des Zeitplans der Schritte der ersten Komponente (704; 804; 904), mit tA(Ereignisse 1 bis n), und des Erzeugens (801) des Zeitplans der Schritte der zweiten Komponente (704; 804; 904), mit tB(Ereignisse 1 bis n), Verwenden der Formel tB(Ereignisse 1 bis n) = tA(Ereignisse 1 bis n) - tA1 + tB1 - ((tA2 - tA1) / 2) - (tC2 - (tC1 + tC3) / 2) aufweist.

7. Das Verfahren nach Anspruch 4, das ferner Bestimmen einer Driftrate bzw. DR unter Verwendung der Formel DR = (tB1ᵢ - tB1ᵢ₋₁) / (tA1ᵢ - tA1ᵢ₋₁) aufweist.

8. Das Verfahren nach Anspruch 7, wobei der Schritt des Erzeugens (704) des Zeitplans der Schritte der ersten Komponente (704; 804; 904), mit tA(Ereignisse 1 bis n), und des Erzeugens (704) des Zeitplans der Schritte der zweiten Komponente (704; 804; 904), mit tB(Ereignisse 1 bis n), Verwenden der Formel tB(Ereignisse 1 bis n) = [tA(Ereignisse 1 bis n) - tA1 + tB1 - ((tA2 - tA1) / 2) - (tB2 - (tA1 + tA2) / 2)] × DR aufweist.

9. Das Verfahren nach Anspruch 6, das ferner Bestimmen einer Driftrate bzw. DR unter Verwendung der Formel DR = (tB1ᵢ - tB1ᵢ₋₁) / (tA1ᵢ - tA1ᵢ₋₁) aufweist.

10. Das Verfahren nach Anspruch 9, wobei der Schritt des Erzeugens (704) des Zeitplans der der Schritte ersten Komponente (704; 804; 904), mit tA(Ereignisse 1 bis n), und des Erzeugens (704) des Zeitplans der Schritte der zweiten Komponente (704; 804; 904), mit tB(Ereignisse 1 bis n), Verwenden der Formel tB(Ereignisse 1 bis n) = [tA(Ereignisse 1 bis n) - tA1 + tB1 - ((tA2 - tA1) / 2) - (tC2 - (tC1 + tC3) / 2)] x DR aufweist.

11. Das Verfahren nach Anspruch 1, das ferner zeitlich Planen eines Integrationsstart- und -endzeitpunktes für einen digitalen Röntgendetektor in dem Zeitplan von Schritten der zweiten Komponente (704; 804; 904) aufweist.

12. Das Verfahren nach Anspruch 11, das ferner zeitlich Planen eines Expositionsstart- und -endzeitpunktes für einen digitalen Röntgendetektor im Zeitplan von Schritten der ersten Komponente (704; 804; 904) aufweist.

13. Das Verfahren nach Anspruch 12, das ferner Folgendes aufweist:
Ausführen des Zeitplans von Schritten durch das radiographische Bildgebungssystem;
Pausieren der Ausführung des Zeitplans von Schritten;
Wiederaufnehmen der Ausführung des Zeitplans von Schritten;
Bestimmen einer verstrichenen Zeit zwischen den Schritten des Pausierens und des Wiederaufnehmens; und
zeitlich Neuplanen nicht ausgeführter Schritte in der pausierten Ausführung des Zeitplans von Schritten,
wobei der Schritt des Neuplanens auf einer Driftrate zwischen der Taktzeit der ersten Komponente und der Taktzeit der zweiten Komponente basiert, wobei die Driftrate verwendet wird, um die Zeiten in den neu geplanten, nicht ausgeführten Schritten zu aktualisieren, und wobei die neu geplanten, nicht ausgeführten Schritte in dem Schritt des Wiederaufnehmens der Ausführung des Zeitplans von Schritten verwendet werden.

14. Das Verfahren nach Anspruch 12, das ferner Folgendes aufweist:
Ausführen des Zeitplans von Schritten durch das radiographische Bildgebungssystem;
Anhalten der Ausführung des Zeitplans von Schritten;
erneutes Starten der Ausführung des Zeitplans von Schritten;
Bestimmen einer verstrichenen Zeit zwischen den Schritten des Anhaltens und des erneut Startens; und
zeitlich Neuplanen der Schritte in dem Zeitplan von Schritten,
wobei der Schritt des Neuplanens auf einer Driftrate zwischen der Taktzeit der ersten Komponente und der Taktzeit der zweiten Komponente basiert,
wobei die Driftrate verwendet wird, um die Schritte in dem Zeitplan von Schritten neu zu planen, und wobei die neu geplanten Schritte in dem Schritt des Neustartens verwendet werden.

## Revendications

1. Procédé de capture d'images radiographiques d'un sujet en utilisant un système d'imagerie radiographique, le système d'imagerie radiographique comportant un premier composant et un deuxième composant, le procédé comprenant :
la génération d'une planification d'étapes du premier composant (704 ; 804 ; 904) à exécuter par le premier composant, la planification d'étapes du premier composant (704 ; 804 ; 904) correspondant chacune à un instant d'horloge du premier composant référencée par le premier composant pour déclencher et arrêter chacune des étapes du premier composant (704 ; 804 ; 904) ; et
la génération d'une planification d'étapes du deuxième composant (704 ; 804 ; 904) à exécuter par le deuxième composant, la planification d'étapes du deuxième composant (704 ; 804 ; 904) correspondant chacune à un instant d'horloge du deuxième composant référencé par le deuxième composant pour déclencher et arrêter chacune des étapes du deuxième composant (704 ; 804 ; 904),
dans lequel les étapes de génération de la planification des étapes du premier composant (704 ; 804 ; 904) et de génération de la planification des étapes du deuxième composant (704 ; 804 ; 904) comprennent :
l'envoi (503 ; 602) d'un premier message numérique sur un réseau numérique à partir du premier composant au deuxième composant et l'envoi (505 ; 604) d'un deuxième message numérique sur le réseau numérique à partir du deuxième composant au premier composant ;
la détermination d'un instant tA1 de l'horloge du premier composant lorsque le premier message numérique a été envoyé par le premier composant ;
la détermination d'un instant tB1 de l'horloge du deuxième composant lorsque le premier message numérique a été reçu au niveau du deuxième composant ;
la détermination d'un instant tA2 de l'horloge du premier composant lorsque le deuxième message numérique a été reçu par le premier composant ; et
la génération de la planification des étapes du premier composant (704 ; 804 ; 904) et la génération de la planification des étapes du deuxième composant (704 ; 804 ; 904) sur la base des instants déterminés tA1, tB1 et tA2, de sorte que les étapes du premier composant (704 ; 804 ; 904) et les étapes du deuxième composant (704 ; 804 ; 904) soient exécutées en synchronie.

2. Procédé selon la revendication 1, comprenant en outre la détermination statistique d'un retard de communication entre l'horloge du premier composant et l'horloge du deuxième composant sur la base d'une répétition des étapes d'envoi (503 ; 602) du premier message numérique et d'envoi (505 ; 604) du deuxième message numérique et de détermination d'une pluralité d'instants tA1 _{i→n}, tB1 _{i→n} et tA2_{i→n}.

3. Procédé selon la revendication 2, comprenant en outre la détermination statistique d'une différence de temps d'horloge relative entre l'instant d'horloge du premier composant et l'instant d'horloge du deuxième composant sur la base de la répétition des étapes d'envoi (503 ; 602) du premier message numérique et d'envoi (505 ; 604) du deuxième message numérique et de détermination de la pluralité des heures tA1_{i→n}, tB1 _{i→n} et tA2_{i→n}.

4. Procédé selon la revendication 1, comprenant en outre la répétition des étapes d'envoi (503 ; 602) du premier message numérique et d'envoi (505 ; 604) du deuxième message numérique et de détermination d'une pluralité d'instants tB2_{i→n} de l'horloge du deuxième composant lorsque les deuxièmes messages numériques ont été envoyés par le deuxième composant.

5. Procédé selon la revendication 4, dans lequel l'étape de génération (801) de la planification des étapes du premier composant (704 ; 804 ; 904), tA(events 1..n), et de génération (801) de la planification des étapes du deuxième composant, tB(events 1..n), comporte l'utilisation de la formule tB(events 1..n) = tA(events 1..n) - tA1 + tB1 - ((tA2 - tA1)/2) - (tB2 - (tA1 + tA2)/2).

6. Procédé selon la revendication 1, comprenant en outre la connexion du premier composant et du deuxième composant à un système de surveillance commun, dans lequel le système de surveillance commun effectue les étapes de :
détermination d'un instant tCl de l'horloge du premier composant lorsque le premier message numérique a été envoyé par le premier composant ;
détermination d'un instant tC2 de l'horloge du deuxième composant lorsque le deuxième message numérique a été envoyé par le deuxième composant ; et
détermination d'un instant tC3 de l'horloge du premier composant lorsque le deuxième message numérique a été reçu par le premier composant,
dans lequel l'étape de génération (801) de la planification des étapes du premier composant, tA(events 1.. n), et de génération de la planification des étapes du deuxième composant (704 ; 804 ; 904), tB(events 1..n), comporte l'utilisation de la formule tB(events 1..n) = tA(events 1..n) - tA1 + tBl - ((tA2 - tA1)/2) - (tC2 - (tCl + tC3)/2).

7. Procédé selon la revendication 4, comprenant en outre la détermination d'une vitesse de dérive, DR, en utilisant la formule DR = (tB1ᵢ - tB1 ᵢ₊₁ )/(tA1ᵢ - tA1 ᵢ₊₁ ).

8. Procédé selon la revendication 7, dans lequel l'étape de génération (704) de la planification des étapes du premier composant (704 ; 804 ; 904), tA(events 1..n), et de génération (704) de la planification des étapes du deuxième composant (704 ; 804 ; 904), tB(events 1..n), comporte l'utilisation de la formule tB(events 1..n) = [tA(events 1..n) - tA1 + tB1 - ((tA2 - tA1)/2) - (tB2 - (tA1 + tA2)/2)] x DR.

9. Procédé selon la revendication 6, comprenant en outre la détermination d'une vitesse de dérive, DR, en utilisant la formule DR = (tB1ᵢ - tB1ᵢ₋₁ )/(tA1ᵢ-tA1ᵢ₋₁).

10. Procédé selon la revendication 9, dans lequel l'étape de génération (704) de la planification des étapes du premier composant (704 ; 804 ; 904), tA(events 1..n), et de génération (704) de la planification des étapes du deuxième composant (704 ; 804 ; 904), tB(events 1..n), comporte l'utilisation de la formule tB(events 1..n) = [tA(events 1..n) - tA1 + tB1 - ((tA2 - tA1)/2) - (tC2 - (tCl + tC3)/2)] x DR.

11. Procédé selon la revendication 1, comprenant en outre la planification d'un instant de début et d'un instant de fin d'intégration pour un détecteur radiographique numérique dans la planification des étapes du deuxième composant (704 ; 804 ; 904).

12. Procédé selon la revendication 1, comprenant en outre la planification d'un instant de début et d'un instant de fin d'exposition pour une source de rayons X dans la planification des étapes du premier composant (704 ; 804 ; 904).

13. Procédé selon la revendication 12, comprenant en outre :
le système d'imagerie radiographique exécutant la planification des étapes ;
la suspension de l'exécution de la planification des étapes ;
la reprise de l'exécution de la planification des étapes ;
la détermination d'un temps écoulé entre les étapes de suspension et de reprise ; et
la replanification des étapes non exécutées de l'exécution suspendue de la planification des étapes,
dans lequel l'étape de replanification est basée sur une vitesse de dérive entre l'instant d'horloge du premier composant et l'instant d'horloge du deuxième composant, la vitesse de dérive étant utilisée pour actualiser les instants des étapes non exécutées replanifiées, et dans lequel les étapes non exécutées replanifiées sont utilisées dans l'étape de reprise d'exécution de la planification des étapes.

14. Procédé selon la revendication 12, comprenant en outre :
le système d'imagerie radiographique exécutant la planification des étapes ;
l'arrêt de l'exécution de la planification des étapes ;
le redémarrage de l'exécution de la planification des étapes ;
la détermination d'un temps écoulé entre les étapes d'arrêt et de redémarrage ; et
la replanification des étapes de la planification des étapes,
dans lequel l'étape de replanification est basée sur une vitesse de dérive entre l'instant d'horloge du premier composant et l'instant d'horloge du deuxième composant, la vitesse de dérive étant utilisée pour replanifier les étapes de la planification d'étapes, et dans lequel les étapes replanifiées sont utilisées dans l'étape de redémarrage.
